# EUROPEAN PATENT SPECIFICATION

(11) **EP 1 226 822 B1**
(45) Date of publication and mention of the grant of the patent: **31.08.2011**
(21) Application number: 02250564.8
(22) Date of filing: 28.01.2002
(51) Int. Cl.: A61K 31/401, A61P 1/00, A61P 7/00, A61P 13/12, A61P 15/10, A61P 31/04, A61P 35/00

(54) **Promoter for production of nitric oxide or nitric oxide synthase, and cosmetic or pharmaceutical composition comprising the same**
Förderer der Herstellung von Stickstoffoxyd und Stickstoffoxydsynthetase, kosmetische und pharmazeutische Zusammenstellungen die diese enthalten
Promoteurs de la production de l'oxyde d'azote et de la NO synthéthase, compositions cosmétiques et pharmaceutiques les contenant

(30) Priority: 29.01.2001 JP 2001020446
(43) Date of publication of application: 31.07.2002
(73) Proprietor: Ajinomoto Co., Inc., Tokyo 104-8315 (JP)
(72) Inventor: Sakamoto, Kazutami, c/o Ajinomoto Co., Inc., Kawasaki-shi, Kanagawa-ken 210-0801 (JP); Watanabe, Kunihiko, c/o Ajinomoto Co., Inc., Kawasaki-shi, Kanagawa-ken 210-0801 (JP); Masaki, Hitoshi, Yokaichi-shi, Shiga-ken 527-0046 (JP)
(74) Representative: Nicholls, Kathryn Margaret

(56) References cited:
- EP-A- 0 221 208
- WO-A1-99/13717
- US-A- 4 025 525
- US-A- 5 508 385
- PATENT ABSTRACTS OF JAPAN vol. 007, no. 061 (C-156), 15 March 1983 (1983-03-15) & JP 57 209256 A (HAMARI YAKUHIN KOGYO KK), 22 December 1982 (1982-12-22)
- DATABASE BIOSIS [Online] BIOSCIENCES INFORMATION SERVICE, PHILADELPHIA, PA, US; 1994, MIRZOIAN S A ET AL: "Central vascular and metabolic effects of pyroglutamic acid." XP002249803 Database accession no. PREV199497363031 & EKSPERIMENTAL'NAYA I KLINICHESKAYA FARMAKOLOGIYA, vol. 57, no. 1, 1994, pages 22-24, ISSN: 0869-2092
- FRANCESCONI M ET AL: "Arginine pyroglutamate in the treatment of chronic cerebrovasculopathies" GAZZETTA MEDICA ITALIANA 1982 ITALY, vol. 141, no. 5, 1982, pages 233-236, XP009014959
- XIAO XIAO-QIU ET AL: "L-Pyroglutamic acid protects rat cortical neurons against sodium glutamate-induced injury." ACTA PHARMACOLOGICA SINICA, vol. 20, no. 8, August 1999 (1999-08), pages 733-736, XP009014835 ISSN: 0253-9756
- DATABASE WPI Week 199953, Derwent Publications Ltd., London, GB; Class B05, AN 1999-615518 & JP 11 269034 A (SHISEIDO CO LTD) 05 October 1999
- DATABASE EMBASE [Online] ELSEVIER SCIENCE PUBLISHERS, AMSTERDAM, NL EMB 0007723931, 1964 'A study of the free aminoacids in the serum of liver diseases'

## Description

The present invention relates to a pharmaceutical composition for use in a method of treatment or prevention of poor blood circulation or platelet aggregation, and to the manufacture of such a composition. In addition, the invention relates to a cosmetic composition and method for improving or promoting blood circulation in the skin.

The physiological and pharmacological roles of nitric oxide (nitrogen monoxide, NO) have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like.

Heretofore, inhibitors of the enzymatic activity of NOS have been examined for the purpose of preventing inflammation and tissue injury which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

It is an object of the present invention to find substances which can maintain the production of NO at an appropriate level in a living body and to provide an excellent promoter for the production of nitric oxide in a living body, utilizing the same, and also a cosmetic or pharmaceutical composition for promoting the production of nitric oxide in a living body.

The present inventors have found that arginine in combination with pyrrolidonecarboxylic acid, a pyrrolidonecarboxylic acid salt, or a pyrrolidonecarboxylic acid derivative, have an excellent effect as such substances, and have accomplished the present invention based on these findings.

Accordingly, in a first aspect the present invention relates to the use of arginine and pyrrolidone carboxylic acid, or a salt, acid anhydride, ester or amide thereof for the manufacture of a medicament for the treatment or prevention of poor blood circulation or platelet aggregation.

In a second aspect, the invention relates to a pharmaceutical composition for use in a method of treatment or prevention of poor blood circulation or platelet aggregation comprising arginine and pyrrolidone carboxylic acid, or a salt, acid anhydride, ester or amide thereof.

In a third aspect, the invention relates to a cosmetic method of improving or promoting blood circulation in the skin comprising applying arginine and pyrrolidone carboxylic acid, or a salt, acid anhydride, ester or amide thereof to the skin. Further, in a fourth aspect, the invention relates to a cosmetic composition for improving or promoting blood circulation in the skin comprising arginine and pyrrolidone carboxylic acid, or a salt, acid anhydride, ester or amide thereof, the composition being in the form of a lotion, emulsion, gel, cream or ointment.

Incidentally, it is to be noted that the production of nitric oxide in a living body at an appropriate level with the specific substances of the present invention may be assumed to be due to the fact that they promote the enzymatic activity (or expressed amount) of cNOS but do not promote the enzymatic activity (or expressed amount) of iNOS, and, however, the conceivable mechanisms concerned are not limited thereto. The fact is important that the specific substances of the present invention when administered to a living body can maintain the production of NO at an appropriate level therein, as evidenced by Examples 2-4 given later.

The present invention will be described below in greater detail.

As pyrrolidonecarboxylic acid to be used according to the present invention, D-pyrrolidonecarboxylic acid (D-2-pyrrolidone-5-carboxylic acid) and L-pyrrolidonecarboxylic acid (L-2-pyrrolidone-5-carboxylic acid)) may be mentioned. Moreover, a mixture composed of both the optical isomers at any ratio (including DL-pyrrolidonecarboxylic acid (DL-2-pyrrolidone-5-carboxylic acid)) may be used. Furthermore, pyrrolidonecarboxylic acid to be used according to the present invention may be in the form of a free acid or may be in the form of the salt with arginine, lysine, a metal ion, triethanolamine, or the like. In addition, it may be in the form of an acid anhydride, an ester, or amide.

The combination of arginine and pyrrolidone carboxylic acid, or a salt, acid anhydride, ester or amide thereof, which acts as a promoter for the production of nitric oxide may be presumed to have an effect of increasing the expressed amount of cNOS in a living body and thus promoting the production of nitric oxide, whereas it substantially does not have an effect of increasing the expressed amount of iNOS, as one of the conceivable mechanisms.

Arginine enhances the effect of the pyrrolidone carboxylic acid, salt, acid anhydride, ester, or amide thereof in promoting production of nitric oxide or nitric oxide synthase in a living body. In this case, arginine may be either L-arginine or D-arginine, or may be a mixture of both the optical isomers at any ratio (including DL-arginine).

In the case that the combination of arginine and pyrrolidone carboxylic acid, or a salt, acid anhydride, ester or amide thereof is used by incorporating it into a cosmetic (a cosmetic composition for promoting the production of nitric oxide or nitric oxide synthase in a living body), the cosmetic composition is used for improving or promoting the blood circulation of the skin. More specifically, the promoter is suitable for use as an ingredient of cosmetics for head hair, head skin, face, body, and the like, bath agents, chilblains-preventing agents, or the like.

The amount of arginine and pyrrolidone carboxylic acid, or a salt acid anhydride, ester or amide thereof included in the cosmetic composition is not particularly limited, but is preferably from 0.01 to 20% by weight, more preferably from 0.05 to 10% by weight, and still more preferably from 0.1 to 5% by weight.

The dosage form of such cosmetics may be a lotion, an emulsion, a gel, a cream, an ointment, or the like.

In the case that the combination of arginine and pyrrolidone carboxylic acid, or a salt, acid anhydride, ester or amide thereof is used by incorporating it into a medicine (a pharmaceutical composition for promoting the production of nitric oxide or nitric oxide synthase in a living body), the combination may be used as, e.g., a pharmaceutical ingredient for inhibiting or preventing poor blood circulation or defective blood circulation or a pharmaceutical ingredient for improving or promoting the blood circulation of the skin. Furthermore, it is possible to prepare a composition, a cosmetic, or the like, for clinical use, using the above-mentioned composition.

In a pharmaceutical composition, the content of arginine and pyrrolidone carboxylic acid, or salt, acid anhydride, ester or amide thereof is not particularly limited, but is preferably from 0.01 to 20% by weight, more preferably from 0.05 to 10% by weight, and still more preferably from 0.1 to 5% by weight.

The dosage form of such pharmaceutical compositions may be in the form of a drug for oral administration such as a liquid, a granule, a powder, a capsule, a tablet, or the like, or an injection or the like for intravenous administration or intra-arterial administration.

Moreover, the above-mentioned composition may be used as an external preparation, e.g., in combination with a substance effective for percutaneous absorption. In the case of using the pharmaceutical composition as an external preparation, such a composition may contain an oil and fat, a wax, a hydrocarbon, an aliphatic acid, a lower alcohol, a higher alcohol, a polyhydric alcohol, an ester, a surfactant, a water soluble polymer, or the like, which is usually used as a base for external preparations. Furthermore, other dermal cell activator, an antiinflammatory agent, an active oxygen eraser, a moisturizing agent, a UV absorber, an antiseptic and antimold agent, a perfume, or the like may be contained.

The dosage form of the external preparation may be any of a lotion, an emulsion, a gel, a cream, an ointment, and the like.

In the following will be described the present invention in further detail with reference to Examples.

### Comparative Examples 1 to 3 Reference Example 1 and Examples 2 to 4:

Angioendothelial cells were cultured using a DMEM medium (Dulbecco-modified MEM) containing 10% fetal bovine serum (FBS). However, phenol red and L-arginine were not added to the medium. The cells were cultured in the medium for 24 hours. After 24 hours of the culture, L-2-pyrrolidone-5-carboxylic acid and L-arginine were added to the medium, the amounts to be added being different for each Example, Reference Example and Comparative Example, as shown in Table 1 below. The culture was further continued for 24 hours.

Thereafter, the produced amount of the nitric oxide was determined by measuring the nitrogen dioxide in the culture supernatant. Nitrogen dioxide was measured by Griess method. The number of the cells in the culture liquid were measured and the formed amount of the nitric oxide per 10³ cells was expressed in terms of nmol. By the way, as negative controls, the example wherein neither arginine nor pyrrolidonecarboxylic acid was contained (Comparative Example 1) and the example wherein arginine was contained singly (Comparative Example 2) were used. Moreover, as a positive control, the example wherein arginine and glycolic acid were contained (Comparative Example 3) was used.

The results are also shown in Table 1. In the table, L-Arg, L-PCA, and GA mean L-arginine, L-pyrrolidonecarboxylic acid, and glycolic acid, respectively.

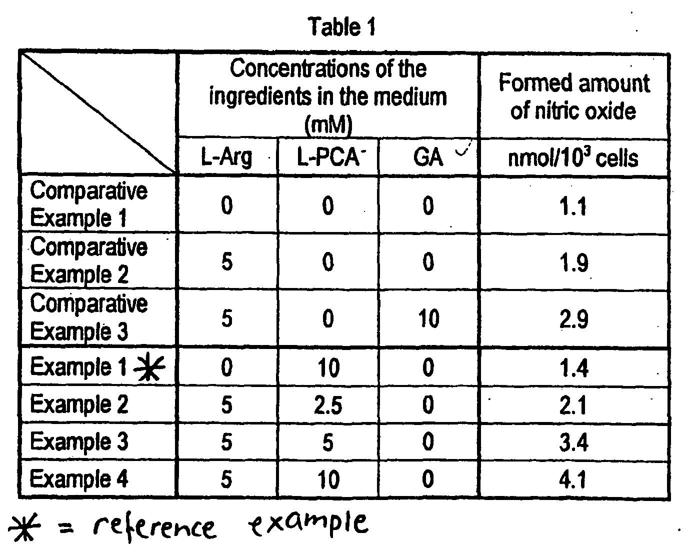

From the results shown in Table 1, in the case that both of pyrrolidonecarboxylic acid and arginine were added to the medium, it is evident that the produced amount of nitric oxide was increased much more than in the case of the combination of glycolic acid and arginine, which combination was hitherto known to have a promoting effect on the production of nitric oxide, when both cases were compared at the same concentration of the added compound(s) (10 mM). Furthermore, it is clear that pyrrolidonecarboxylic acid alone exhibited a promoting effect on the production of nitric oxide and the produced amount of nitric oxide was increased as the added amount was increased.

### Example 5 (Reference)

Angioendothelial cells were cultured using a DMEM medium (Dulbecco-modified MEM) containing 10% fetal bovine serum (FBS). The cells were cultured in the medium for 24 hours. After 24 hours of the culture, the cells were further cultured in the medium to which L-pyrrolidonecarboxylic acid was added so as to achieve a predetermined concentration (See Table 3 given later) for 24 hours, and then the RNA was extracted. For reverse transcription of the RNA, a kit for reverse transcription (manufactured by GIBCO) was used to prepare the cDNA. The cDNA was then subjected to PCR (RT-PCR) . As primers, those shown in Table 2 below were used. Forty cycles of the PCR were carried out under the conditions of denaturing at 95°C for 30 seconds, annealing at 59°C for 60 seconds, and extension at 73°C for 90 seconds.

**Table 2**

| | | |
|---|---|---|
| cNOS | sense | 5'-GTG ATG GCG AAG CGA GTG AAG-3' |
| | anti-sense | 5'-CCG AGC CCG GGC GCG CAG AAC-3' |
| iNOS | sense | 5'-TTG GAG GCA AAC AGC ACA TTC A-3' |
| | anti-sense | 5'-GGG TTG GGG GTG TGG TGA TGA TGT-3' |

As has been described above, using RT-PCR, the expressed amounts of the messenger RNA's of cNOS and iNOS were determined. In this experiment, cNOS was detected, but iNOS was not detected. Furthermore, it was recognized that the amount of cNOS tended to increase depending on the increasing amount of pyrrolidonecarboxylic acid added, as shown in Table 3 below. In the table, L-PCA means L-pyrrolidonecarboxylic acid.

**Table 3**

| Concentration of L-PCA added (mM) | Expressed amount of cNOS (Relative ratio) |
|---|---|
| 1.0 | 1.00 |
| 2.5 | 1.25 |
| 5.0 | 1.40 |
| 10.0 | 1.75 |

By the way, with regard to pyrrolidonecarboxylic acid and arginine, experiments were carried out in the case that D-isomer was used instead of L-isomer, in the case that both isomers were combined, and in the like, whereby similar results were obtained.

### Example 6: O/W type emulsion

An O/W type emulsion was prepared, using the following composition of raw materials and by the method described below.

Composition of raw materials; (1) squalane: 5.0 (% by weight, the same in the following), (2) white vaseline: 2.0, (3) beeswax: 0.5, (4) sorbitan sesquioleate: 0.8, (5) polyoxyethylene oleyl ether (20 EO): 1.2, (6) methyl p-oxybenzoate: 0.1, (7) propylene glycol: 5.0, (8) purified water: 57.1, (9) carboxyvinyl polymer (aqueous 1.0% by weight solution): 20.0, (10) potassium hydroxide: 0.1, (11) ethanol: 5.0, (12) L-pyrrolidonecarboxylic acid-L-arginine salt (aqueous 10% by weight solution) : 3.0, and (13) a perfume: 0.2.

Preparation method; The oily phase ingredients (1) to (5) were mixed and heated to 75°C, whereby the whole was melted and homogenized. On the other hand, the aqueous phase ingredients (6) to (8) were mixed and dissolved, followed by heating to 75°C. Thereto were added the above melted and homogenized oily phase ingredients, followed by pre-emulsification. The ingredient (9) was added to the pre-emulsified mass, and then the resulting mixture was homogeneously emulsified with a homomixer. Then, thereto was further added the ingredient (10) to adjust the pH. After cooling, the ingredients (11) to (13) were added thereto at 40°C, and the whole was mixed and homogenized.

A use experiment was carried out on one group composed of 10 members of 20's to 40's females, who worry about the black rings beneath their eyes (5 members of them having a subjective symptom of anemia), using the above-prepared emulsion.
Furthermore, a comparative experiment was carried out on another group composed of similar members using the same emulsion except that the raw material (12), i.e., L-pyrrolidonecarboxylic acid-L-arginine salt was replaced by water (Comparative Example 4). The two kinds of emulsions were used in the respective groups, twice a day for 2 months by applying one of them to the black rings beneath the eyes. The improvement of the black rings was investigated after 2 weeks and after 2 months.

Concerning the test subjects who used the emulsion of the present invention, the black rings were all improved after 2 months, and especially concerning the test subjects who had a symptom of anemia, the black rings were improved only after 2 weeks. On the contrary, in the case of the emulsion of Comparative Example 4 wherein L-pyrrolidonecarboxylic acid-arginine salt was replaced by water, no significant improvement of the black rings was observed.

By the way, with regard to the two kinds of emulsions in the present Example 6, there were not observed any conditional changes of the preparations, such as precipitation, separation, or agglomeration of the ingredients, smell change, or color change, during the above use test period. Moreover, both in the group using the composition of the present invention and the group using the composition of Comparative Example, there were no test subjects who exhibited skin irritation or skin sensitization reaction.

### Example 7: Lotion

A lotion was prepared by mixing and homogenizing (1) ethanol: 10.0 (% by weight, the same in the following), (2) hydroxyethyl cellulose: 1.0, (3) L-pyrrolidonecarboxylic acid-L-arginine salt (aqueous 10% by weight solution) : 3.0, (4) methyl p-oxybenzoate: 0.1, and (5) purified water: 85.9.

### Example 8: O/W type emulsion

An O/W type emulsion was prepared, using the following composition of raw materials and by the method described below.

Composition of raw materials; (1) stearic acid: 0.2 (% by weight, the same in the following), (2) cetanol: 1.5, (3) vaseline: 3.0, (4) liquid paraffin: 7.0, (5) polyoxyethylene (10 EO) mono-oleic acid ester: 1.5, (6) tocopherol acetate: 0.5, (7) glycerol: 5.0, (8) methyl p-oxybezoate: 0.1, (9) triethanolamine: 1.0, (10) purified water: 76.2, and (11) L-pyrrolidonecarboxylic acid-L-arginine salt (aqueous 10% by weight solution): 4.0.

Preparation method; The oily phase ingredients (1) to (6) were mixed and heated to 70°C, whereby the whole was melted and homogenized. The resulting mass was, as it was, maintained at the same temperature. On the other hand, the aqueous phase ingredients (7) to (10) were mixed and homogenized by heating to 70°C. Then, thereto was gradually added with stirring, the above-mentioned oily phase ingredients to emulsify. The resulting emulsion was cooled and added with the ingredient (11) at 40°C, followed by mixing.

### Example 9: Aqueous gel

An aqueous gel was prepared, using the following composition of raw materials and by the method described below.

Composition of raw materials; (1) purified water: 84.3 (% by weight, the same in the following), (2) carboxyvinyl polymer: 0.5, (3) dipropylene glycol: 10.0, (4) methyl p-oxybenzoate: 0.1, (5) potassium hydroxide: 0.1, and (6) L-pyrrolidonecarboxylic acid-DL-arginine salt (aqueous 10% by weight solution): 5.0.

Preparation method; The ingredient (2) was homogeneously dissolved in the ingredient (1), and thereto was added the ingredient (4) dissolved in the ingredient (3). Thereafter, the viscosity was increased by adding the ingredient (5), and finally, the ingredient (6) was added thereto.

### Example 10: W/O emulsion type ointment

A W/O emulsion type ointment was prepared, using the following composition of raw materials and by the method deseribed below.

Composition of raw materials; (1) liquid paraffin: 30.0 (% by weight, the same in the following), (2) microcrystalline wax: 2.0, (3) vaseline; 5.0, (4) diglycerol oleic acid ester: 5.0, (5) propylene glycol: 3.0, (6) methyl p-oxybenzoate: 0.1, (7) L-pyrrolidonecarboxylic acid-L-arginine salt: 0.5, and (8) purified water: 54.4.

Preparation method; To the ingredients (1) to (4) which had been mixed and melted by heating to 70°C was gradually added the ingredients (5) to (8) which had been dissolved and homogenized by similarily heating. The resulting mass was homogenized and cooled with stirring.

According to the present invention, there may be easily provided an excellent promoter for the production of nitric oxide or nitric oxide synthase in a living body, and, in turn, a cosmetic or pharmaceutical composition for promoting the production of nitric oxide or nitric oxide synthase in a living body, using the said promoter.

## Claims

1. Use of arginine and pyrrolidone carboxylic acid, or a salt, acid anhydride, ester or amide thereof for the manufacture of a medicament for the treatment or prevention of poor blood circulation or platelet aggregation.

2. Use according to claim 1 wherein the medicament is for improving or promoting blood circulation in the skin.

3. Use according to claim 1 or claim 2 wherein the medicament is for intravenous or intra-arterial administration.

4. Use according to claim 1 or claim 2 wherein the medicament is for external use.

5. A use according to any one of claims 1 to 4 wherein the arginine and pyrrolidone carboxylic acid are incorporated into the medicament as a pyrrolidone carboxylic acid - arginine salt.

6. A pharmaceutical composition for use in a method of treatment or prevention of poor blood circulation or platelet aggregation comprising arginine and pyrrolidone carboxylic acid, or a salt, acid anhydride, ester or amide thereof.

7. A pharmaceutical composition according to claim 6 for the use according to claim 6 in the form of a liquid, granule, powder, capsule, or tablet for oral administration.

8. A pharmaceutical composition according to claim 6 for the use according to claim 6 for intravenous or intra-arterial administration.

9. A pharmaceutical composition according to claim 6 for the use according to claim 6 for external use.

10. A pharmaceutical composition according to claim 6 for the use according to any one of claims 6 to 9 comprising arginine and pyrrolidone carboxylic acid in the form of a pyrrolidone carboxylic acid - arginine salt.

11. A cosmetic method of improving or promoting blood circulation in the skin comprising applying arginine and pyrrolidone carboxylic acid, or a salt, acid anhydride, ester or amide thereof to the skin.

12. A cosmetic composition for improving or promoting blood circulation in the skin comprising arginine and pyrrolidone carboxylic acid, or a salt, acid anhydride, ester or amide thereof, the composition being in the form of a lotion, emulsion, gel, cream or ointment.

13. A cosmetic composition according to claim 12 containing from 0.01 to 20% by weight arginine and pyrrolidone carboxylic acid, or a salt, acid anhydride, ester or amide thereof.

14. A cosmetic composition according to claim 12 or 13 comprising arginine and pyrrolidone carboxylic acid in the form of a pyrrolidone carboxylic acid - arginine salt.

## Patentansprüche

1. Verwendung von Arginin und Pyrrolidoncarbonsäure oder einem Salz, Säureanhydrid, Ester oder Amid davon zur Herstellung eines Medikaments zur Behandlung oder Prävention von schwachem Blutkreislauf oder Plättchenaggregation.

2. Verwendung nach Anspruch 1, worin das Medikament der Verbesserung oder Förderung des Blutkreislaufs in der Haut dient.

3. Verwendung nach Anspruch 1 oder Anspruch 2, worin das Medikament der intravenösen oder intraarteriellen Verabreichung dient.

4. Verwendung nach Anspruch 1 oder Anspruch 2, worin das Medikament der äußerlichen Anwendung dient.

5. Verwendung nach einem der Ansprüche 1 bis 4, worin das Arginin und die Pyrrolidoncarbonsäure in das Medikament als Pyrrolidoncarbonsäure-Arginin-Salz eingearbeitet sind.

6. Pharmazeutische Zusammensetzung zur Verwendung in einem Verfahren zur Behandlung oder Prävention von schwachem Blutkreislauf oder Plättchenaggregation, die Arginin und Pyrrolidoncarbonsäure oder ein Salz, ein Säureanhydrid, einen Ester oder ein Amid davon umfasst.

7. Pharmazeutische Zusammensetzung nach Anspruch 6 zur Verwendung nach Anspruch 6 in Form einer Flüssigkeit, eines Körnchens, eines Pulvers, einer Kapsel oder einer Tablette zur oralen Verabreichung.

8. Pharmazeutische Zusammensetzung nach Anspruch 6 zur Verwendung nach Anspruch 6 zur intravenösen oder intraarteriellen Verabreichung.

9. Pharmazeutische Zusammensetzung nach Anspruch 6 zur Verwendung nach Anspruch 6 zur äußerlichen Anwendung.

10. Pharmazeutische Zusammensetzung nach Anspruch 6 zur Verwendung nach einem der Ansprüche 6 bis 9, die Arginin und Pyrrolidoncarbonsäure in Form eines Pyrrolidoncarbonsäure-Arginin-Salzes umfasst.

11. Kosmetisches Verfahren zur Verbesserung oder Förderung des Blutkreislaufs in der Haut, umfassend das Auftragen von Arginin und Pyrrolidoncarbonsäure oder einem Salz, Säureanhydrid, Ester oder Amid davon auf die Haut.

12. Kosmetische Zusammensetzung zur Verbesserung oder Förderung des Blutkreislaufs in der Haut, umfassend Arginin und Pyrrolidoncarbonsäure oder ein Salz, ein Säureanhydrid, einen Ester oder ein Amid davon, wobei die Zusammensetzung in Form einer Lotion, einer Emulsion, eines Gels, einer Creme oder einer Salbe vorliegt.

13. Kosmetische Zusammensetzung nach Anspruch 12, enthaltend 0,01 bis 20 Gew.-% Arginin und Pyrrolidoncarbonsäure oder ein Salz, ein Säureanhydrid, einen Ester oder ein Amid davon.

14. Kosmetische Zusammensetzung nach Anspruch 12 oder 13, umfassend Arginin und Pyrrolidoncarbonsäure in Form eines Pyrrolidoncarbonsäure-Arginin-Salzes.

## Revendications

1. Utilisation d'arginine et d'acide pyrrolidone-carboxylique, ou d'un sel, d'un anhydride d'acide, d'un ester ou d'un amide de ceux-ci pour la fabrication d'un médicament destiné au traitement ou à la prévention d'une circulation sanguine médiocre ou de l'agrégation de plaquettes.

2. Utilisation selon la revendication 1, dans laquelle le médicament est destiné à améliorer ou à promouvoir la circulation sanguine dans la peau.

3. Utilisation selon la revendication 1 ou la revendication 2, dans laquelle le médicament est destiné à une administration intraveineuse ou intra-artérielle.

4. Utilisation selon la revendication 1 ou la revendication 2, dans laquelle le médicament est destiné à un usage externe.

5. Utilisation selon l'une quelconque des revendications 1 à 4, dans laquelle l'arginine et l'acide pyrrolidone-carboxylique sont incorporés dans le médicament comme un sel d'acide pyrrolidone-carboxylique-arginine.

6. Composition pharmaceutique pour une utilisation dans un procédé de traitement ou de prévention d'une circulation sanguine médiocre ou de l'agrégation de plaquettes comprenant de l'arginine et de l'acide pyrrolidone-carboxylique, ou un sel, un anhydride d'acide, un ester ou un amide de ceux-ci.

7. Composition pharmaceutique selon la revendication 6 destinée à l'utilisation selon la revendication 6 dans la forme d'un liquide, d'un granulé, d'une poudre, d'une capsule ou d'un comprimé pour une administration orale.

8. Composition pharmaceutique selon la revendication 6 destinée à l'utilisation selon la revendication 6 pour une administration intraveineuse ou intra-artérielle.

9. Composition pharmaceutique selon la revendication 6 destinée à l'utilisation selon la revendication 6 pour un usage externe.

10. Composition pharmaceutique selon la revendication 6 destinée à l'utilisation selon l'une quelconque des revendications 6 à 9 comprenant de l'arginine et de l'acide pyrrolidone-carboxylique dans la forme d'un sel d'acide pyrrolidone-carboxylique-arginine.

11. Procédé cosmétique pour l'amélioration ou la promotion de la circulation sanguine dans la peau comprenant l'application d'arginine et d'acide pyrrolidone-carboxylique, ou d'un sel, d'un anhydride d'acide, d'un ester ou d'un amide de ceux-ci sur la peau.

12. Composition cosmétique destinée à améliorer ou à promouvoir la circulation sanguine dans la peau comprenant de l'arginine et de l'acide pyrrolidone-carboxylique, ou un sel, un anhydride d'acide, un ester ou un amide de ceux-ci, la composition étant dans la forme d'une lotion, d'une émulsion, d'un gel, d'une crème ou d'un baume.

13. Composition cosmétique selon la revendication 12 contenant de 0,01 à 20 % en masse d'arginine et d'acide pyrrolidone-carboxylique, ou d'un sel, d'un anhydride d'acide, d'un ester ou d'un amide de ceux-ci.

14. Composition cosmétique selon la revendication 12 ou 13 comprenant de l'arginine et de l'acide pyrrolidone-carboxylique dans la forme d'un sel d'acide pyrrolidone-carboxylique-arginine.
